# EUROPÄISCHE PATENTSCHRIFT

(11) **EP 0 714 304 B1**
(45) Veröffentlichungstag und Bekanntmachung des Hinweises auf die Patenterteilung: **07.06.2000**
(21) Anmeldenummer: 94924861.1
(22) Anmeldetag: 10.08.1994
(51) Int. Cl.: A61K 31/59, A61K 9/70

(54) **THERAPEUTISCHES SYSTEM ZUR BEHANDLUNG DER PSORIASIS**
THERAPEUTIC SYSTEM FOR TREATING PSORIASIS
SYSTEME THERAPEUTIQUE POUR LE TRAITEMENT DU PSORIASIS

(30) Priorität: 21.08.1993 DE 4328217
(43) Veröffentlichungstag der Anmeldung: 05.06.1996
(73) Patentinhaber: LTS Lohmann Therapie-Systeme AG, 56626 Andernach (DE)
(72) Erfinder: LIST, Harald, D-56567 Neuwied (DE)
(74) Vertreter: Flaccus, Rolf-Dieter, Dr.
(86) Internationale Anmeldenummer: EP9402650
(87) Internationale Veröffentlichungsnummer: WO9505829

(56) Entgegenhaltungen:
- EP-A- 0 549 318
- WO-A-89/10351
- PHARM. ZTG. Bd. 138, Nr. 28 , 1993 Seiten 32 - 36 BARBARA PERUCHE 'Calcipotriol, ein neues Antipsoriatikum'
- Römpp's Chemielexikon, Seiten 548 und 3622
- W.Schunack et al., Arzneistoffe, 1981, Seiten 30-33

## Beschreibung

Gegenstand der vorliegenden Erfindung ist ein therapeutisches System zur Verabreichung von Wirkstoffen für die Behandlung von Hauterkrankungen mit starker Zellproliferation.

Eine große Zahl von Menschen - allein für Deutschland nimmt man circa 2 Millionen Betroffene an - leidet unter Schuppenflechte (Psoriasis) unterschiedlicher Schweregrade. Damit gehört die Psoriasis zu den häufigsten Hauterkrankungen. Ursache für diese Hauterkrankung ist eine genetische Veränderung, welche die Immunabwehr des Organismus beeinflußt. Deshalb ist bis heute auch keine Heilungsmöglichkeit gegeben, jedoch stehen Behandlungsverfahren zur Verfügung, die deutliche Linderung verschaffen können.

Dazu gehören vor allem externe Anwendungen mit der Applikation von Wirkstoffen, welche die beschleunigte Zellvermehrung mit Schuppenbildung reduzieren sollen, z. B. Präparate aus Steinkohlenteer, Salicylsäure, Dithranol, Cortison, Fumarsäurederivate. Ein weiteres therapeutisches Verfahren ist die Photochemotherapie. Auch Okklusivverbände tragen zur Besserung bei. Darüber hinaus werden bei schweren Formen der Psoriasis innerlich wirkende Präparate wie Glucocorticoide, Vitamin A-Derivate, Zytostatika (Methotrexat) oder Immuntherapeutika (Cyclosporin A) eingesetzt.

Allen diesen Verfahren, über die zahlreiche Publikationen vorliegen, haften Nachteile und Nebenwirkungen an wie
1. Nephrotoxizität
2. Embryotoxizität
3. gastrointestinale Nebenwirkungen
4. Rebound-Effekte

Manche lokal einzusetzende Mittel bedürfen einer zusätzlichen Abdeckung, um eine Verschmutzung der Kleidung zu vermeiden.

Seit einigen Jahren ist bekannt, daß das natürlich vorkommende Vitamin D 3 ebenfalls in der Lage ist, die Psoriasis zu beeinflussen: In Zellkulturen hemmte Vitamin D 3 die Zellproliferation und induzierte die Ausdifferenzierung der Zellen.

Bei der für eine signifikante Wirksamkeit notwendigen Dosierung zeigten sich jedoch sowohl bei der oralen als auch bei der cutanen Anwendung erhebliche Beeinträchtigungen des Calciumstoffwechsels. Ein therapeutischer Einsatz bei der Psoriasis war deshalb nicht möglich. Es wurden daher Vitamin D 3-Derivate synthetisiert mit dem Ziel die durch Vitamin D 3 bedingten Nebenwirkungen Hypercalcämie, Knochenresorption und Hypercalciurie zu vermeiden oder zumindest stark zu reduzieren. Vitamin D 3-Derivate wie z.B. Calcitriol, die der Regulierung des Calciumstoffwechsels dienen, sind daher im Sinne der vorliegenden Erfindung nicht verwendbar. Die Synthese weiterer Vitamin D 3-Analoga ist in EP 0 227 826 und WO 89/10 351) beschrieben.

Auch CALCIPOTRIOL der nachstehenden Formel: 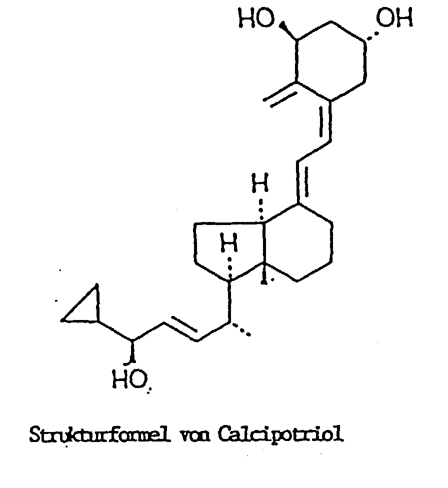 wurde bereits sythetisiert und als Wirkstoff zur Behandlung von Psoriasis untersucht (Pham.Ztg. 138 (28) Seite 32 ff.)

Dabei wurde gefunden, daß das synthetische 1,24-Dihydroxy--Derivat des Vitamins D 3 (INN CALCIPOTRIOL) bei der Induktion der Differenzierung und Proliferationshemmung in Keratinozytenkulturen ebenso wirksam ist, wie Vitamin D 3 selbst; dabei beträgt die nachteilige Beeinflussung des Calciumstoffwechsels aber weniger als ein Hundertstel derjenigen des Vitamin D 3.

Zu dieser mit dem Prioritätsdatum der vorliegenden Anmeldung etwa zeitgleichen Veröffentlichung wird die kleinflächige äußerliche Anwendung von Calcipotriol in Salbenform bei leichter und mittelschwerer Psoriasis beschrieben, wobei auf Störungen (wie Jucken, Brennen, Erytheme) besonders hingewiesen wird. Die Salbe soll zweimal täglich und in einer Menge von maximal 15 g/Tag auf nicht mehr als 30 % der Hautoberfläche unter wiederholter Kontrolle des Serumcalciumspiegels erfolgen, offenbar um ein Entgleiden des Serumcalciumspiegels nach oben zu vermeiden.

Diese Nachteile werden durch das erfindungsgemäße therapeutische System gemäß Patentanspruch 1 vermieden, mit dem eine Darreichungsform bereitgestellt wird, welche die Effektivität des Wirkstoffes steigert, d.h. die Dosis/Wirkungsrelation verbessert, Nebenwirkungen weiter reduziert bzw. vermeidet und ein für den Anwender akzeptables Applikationsverfahren ermöglicht.

Ein therapeutisches System ist eine arzneistoffenthaltende Vorrichtung bzw. eine Darreichungsform, die einen oder mehrere Arzneistoffe in vorausbestimmter Rate kontinuierlich über einen festgelegten Zeitraum an einen festgelegten Anwendungsort abgibt (zitiert nach HEILMANN "Therapeutische Systeme", Ferdinand-Enke-Verlag, Stuttgart 1984).

Das Calcipotriol gehört zu den Calciferolen, die für eine in vito Umwandlung der entsprechenden Vorstufen (insbesondere durch Lichteinwirkung oder Hydroxylierung) in den eigentlichen Wirkstoff bekannt sind. Demgemäß kann selbstverständlich das Calcipotriol in Form eines Prodrugs im System vorgesehen werden.

Die Anwendung eines therapeutischen Systems bedeutet für die Verabreichung von Wirkstoffen aus der Gruppe der Vitamin D 3-Derivate, daß die Intervalle der Applikation, insbesondere der lokalen, verlängert werden können, daß die kontrollierte Wirkstoffliberation Resorptionsspitzen mit nachfolgender systemischer Wirkung vermeidet, die Behandlung größerer Hautflächen ermöglicht wird und die bisher limitierte Behandlungsdauer, auch mit Unterbrechungen, verlängert werden kann - ein ganz wichtiger Punkt hinsichtlich der lebenslangen Behandlungsbedürftigkeit.

Darüber hinaus bedeutet ein derartiges therapeutisches System gleichzeitig eine sinnvolle Abdeckung der behandelten Hautareale, die je nach Ausgestaltung bis zu mehreren Tagen belassen werden kann. Das therapeutische System wirkt im Sinne der aus der Literatur bekannten positiven Auswirkung der Okklusion, und die Hygienebedürfnisse der Patienten können befriedigend gelöst werden. Die Applikation erfolgt nach zuschneiden auf Form und Größe der betroffenen Hautareale.

Die im Rahmen der Erfindung vorgesehenen therapeutischen Systeme in Pflasterform können prinzipiell vorliegen als:
1. Membrankontrollierte Systeme
2. Matrixkontrollierte Systeme

Für Pflaster mit Membran-Steuerung sei beispielhaft auf die DE-A 21 35 533 hingewiesen. Diese Pflaster bestehen grundsätzlich aus einer Rückschicht, die eine der Oberflächen darstellt, einer für den Wirkstoff durchlässigen Klebeschicht, die die andere Oberfläche darstellt und letztlich einem Reservoir, das den Wirkstoff zwischen den beiden die Oberflächen bildenden Schichten enthält.

Alternativ dazu kann der Wirkstoff auch in einer Vielzahl von Mikrokapseln enthalten sein, die innerhalb der durchlässigen Klebschicht verteilt sind.

In jedem Fall wird der Wirkstoff aus dem Reservoir oder den Mikrokapseln durch eine Membran in die für den Wirkstoff durchlässige Klebschicht, die im Kontakt mit der Haut des zu Behandelnden steht, kontinuierlich abgegeben. Im Falle von Mikrokapseln kann das Kapselmaterial auch als Membran wirken.

Ein Pflaster mit Matrix-Diffusions-Steuerung wird z. B. in DE-PS 33 15 272 beschrieben. Es besteht aus einer undurchlässigen Rückschicht, einem damit verbundenen, besonders aufgebauten Reservoir aus einer Polymermatrix, das den Wirkstoff in einer Konzentration oberhalb der Sättigungskonzentration enthält, einer mit dem Reservoir verbundenen, für den Wirkstoff durchlässigen Haftklebeschicht und einer die Haftklebeschicht abdeckenden, zum Gebrauch wieder ablösbaren Schutzschicht. Ist die Reservoirmatrix selbst schon haftklebend, so kann auf die zusätzliche Haftklebeschicht verzichtet werden.

Die Dosierung von CALCIPOTRIOL muß so gewählt werden, daß Abgaberaten erreicht werden, die in ihrer therapeutischen Effizienz der Salbenapplikation gleichwertig sind. Dabei ist zu berücksichtigen, daß das therapeutische System gegebenenfalls auch über mehrere Tage belassen werden kann.

Vorteilhaft geben diese Systeme zwischen 0,05 und 5,0 Mikrogramm CALCIPOTRIOL pro cm² und 24 h ab.

Die beschriebenen therapeutischen Systeme werden zur Herstellung eines gebrauchsfertigen Arzneimittels verwendet. Dazu bedarf es der Festlegung von Parametern wie z. B. Wirkstoffwahl, Dosis, Steuerung der Freisetzung und Freisetzungsrate, Zusammensetzung des Reservoirs und eventuellen Zusatz von Hilfsstoffen. So kann der Zusatz von Permeationsbeschleunigern, z.B. DMSO oder AZON^{R} zur Steigerung der Resorption in tieferliegende Hautschichten sinnvoll Sein. Aus dem gleichen Grund kann auch ein weiteres Prinzip zur Verbesserung der Permeation von Wirkstoffen berücksichtigt werden, nämlich die Verwendung von elektrischem Strom (Iontophorese).

CALCIPOTRIOL kann in unterschiedlicher Form in ein therapeutisches System eingebracht werden, es kann auch mikroverkapselt vorliegen. Schließlich kann es sinnvoll sein, CALCIPOTRIOL mit anderen wirkstoffen zu kombinieren - mit dem Ziel einer Wirkungssteigerung, gegebenenfalls aber auch Reduzierung der Einzeldosen. Kombinationen sind möglich mit z.B. Cortison, Alpha-Tocopherol, Acetylsalicylsäure und/oder Fumarsäure. Vorteilhafterweise enthält das therapeutische System ein wirkstoffhaltiges Reservoir auf der Basis einer Gel- bzw. Kolloidschicht, z.B. in Form eines Polyacrylat-Hydrogels oder eines Hydrokolloids aus Gelatine, Pektin oder Carboxymethylcellulose.

Zusammenfassend können mit dem beschriebenen therapeutischen System, welches als Wirkstoff CALCIPOTRIOL enthält. bei der Behandlung von Hauterkrankungen mit abnormaler Zellproliferation und/oder gestörter Zelldifferenzierung die folgenden Vorteile erzielt werden:
- kontrollierte, konstante Wirkstofffreisetzung mit der Vermeidung systemischer Verfügbarkeit
- verbesserte Dosis/Wirkungsrelation, Vermeiden der für die übliche, topische Anwendung geltenden Einschränkungen hinsichtlich der Behandlungsdauer und Ausdehnung der zu behandelnden Hautareale
- optimierte Behandlung auch hinsichtlich des therapeutischen Systems mit gleichzeitiger Schutzfunktion, erhöhte Akzeptanz beim Anwender.

## Patentansprüche

1. Therapeutisches System zur Verabreichung von Wirkstoffen für die Behandlung von Hauterkrankungen mit starker Zellproliferation, **dadurch gekennzeichnet**, daß es in Pflasterform vorliegt und eine Rückschicht, ein damit verbundenes Wirkstoffreservoir aus einer Polymermatrix, bei Abwesenheit anderer Steuermechanismen eine die Abgabe des Wirkstoffs steuernde Mambran, eine Haftklebeeinrichtung zur Befestigung des Systems auf der Haut und im Bedarfsfall eine vor Appliaktion des Systems ablösbare Schutzschicht umfaßt, und daß es als Wirkstoff CALCIPOTRIOL selbst oder als Prodrug enthält.

2. Therapeutisches System nach Anspruch 1**, dadurch gekenn****zeichnet**, daß es zwischen 0,05 und 5,0 Mikrogramm CALCIPOTRIOL pro cm² und 24 h abgibt.

3. Therapeutisches System nach Anspruch 1 oder 2, **dadurch gekennzeichnet**, daß es eine hochflexible, wasserdampfdurchlässige Polymerfolie als Rückschicht enthält.

4. Therapeutisches System nach Anspruch 3, **dadurch gekennzeichnet**, daß das Polymer ein Polyurethan ist.

5. Therapeutisches System nach einem oder mehreren der Ansprüche 1 bis 4, **dadurch gekennzeichnet**, daß das Wirkstoffreservoir mit Hilfe einer Gel- oder Kolloidschicht gebildet ist.

6. Therapeutisches System nach einem oder mehreren der Ansprüche 1 bis 5, **dadurch gekennzeichnet**, daß die Diffusion des Wirkstoffes aus dem Reservoir oder die Permeation des Wirkstoffes in die Haut durch den Zusatz von geeigneten Stoffen beeinflußbar ist.

7. Therapeutisches System nach einem oder mehreren der Ansprüche 1 bis 6, **dadurch gekennzeichnet**, daß eine Kombination von Calcipotriol bzw. Calcipotriol-Prodrug mit anderen Wirkstoffen eingesetzt wird.

8. Therapeutisches System nach Anspruch 7, **dadurch gekennzeichnet**, daß eine Kombination des Wirkstoffs mit Cortison, Alpha-Tocopherol, Acetylsalicylsäure und/oder Fumarsäure vorliegt.

9. Verfahren zur Herstellung eines therapeutischen Systems gemäß einem oder mehreren der Ansprüche 1 bis 8, **dadurch gekennzeichnet**, daß das CALCIPOTRIOL bzw. sein Prodrug in fester Form, in Lösung oder in Dispersion in das Verabreichungssystem eingebracht wird, wobei übliche Hilfsstoffe zugesetzt werden können.

10. Verwendung eines therapeutischen Systems nach einem oder mehreren der Ansprüche 1 bis 9 zur Herstellung eines gebrauchsfertigen Arzneimittels zur Therapie von Hauterkrankungen mit starker Zellproliferation, insbesondere der Psoriasis.

## Claims

1. A therapeutic system for the administration of active substances in the treatment of skin diseases involving considerable cell proliferation, characterized in that it is present in the form of a plaster and comprises a backing layer, an active substance reservoir connected thereto and made up of a polymer matrix, where no other control mechanisms are present a membrane controlling the active substance release, a pressure sensitive adhesive device for affixing the system to the skin and, if required, a protective layer which is removable prior to application, and in that it contains as active substance CALCIPOTRIOL itself or as a prodrug.

2. The therapeutic system according to claim 1, characterized in that it releases between 0.05 and 5.0 microgrammes of CALCIPOTRIOL per cm² and 24 h.

3. The therapeutic system according to claim 1 or 2, characterized in that it comprises a highly flexible, water vapour-permeable polymer film or sheet as backing layer.

4. The therapeutic system according to claim 3, characterized in that the polymer is a polyurethane.

5. The therapeutic system according to one or more of claims 1 to 4, characterized in that the active substance reservoir is formed with the aid of a gel layer or colloid layer.

6. The therapeutic system according to one or more of claims 1 to 5, charcterized in that the diffusion of the active substance from the reservoir, or the permeation of the active substance into the skin can be influenced by the addition of suitable substances.

7. The therapeutic system according to one or more of claims 1 to 6, characterized in that a combination of CALCIPOTRIOL or CALCIPOTRIOL prodrug with other active substances is used.

8. The therapeutic system according to claim 7, characterized in that a combination of the active substance with cortisone, alpha-tocopherol, acetylsalicylic acid and/or fumaric acid is present.

9. A process for the production of a therapeutic system according to one or more of claims 1 to 8, characterized in that CALCIPOTRIOL or its prodrug is introduced into the administration system in solid form, in solution or in dispersion, there being the possibility of adding common auxiliary agents.

10. The use of a therapeutic system according to one or more of claims 1 to 9 for the production of a ready-for-use medicament for the therapy of skin diseases involving severe cell proliferation, in particular psoriasis.

## Revendications

1. Système thérapeutique destiné à l'administration de principes actifs pour le traitement de maladies cutanées liées à une forte prolifération cellulaire, caractérisé en ce qu'il est présent sous la forme d'un emplâtre et comprend une couche dorsale, un réservoir de principe actif lié à cette dernière et constitué par une matrice polymère, en l'absence d'autres mécanismes de commande, une membrane commandant le dégagement du principe actif, un mécanisme auto-adhésif pour la fixation du système sur la peau et, en cas de besoin, une couche de protection qui peut être pelliculée avant l'application du système, et en ce qu'il contient à titre de principe actif du CALCIPOTRIOL en tant que tel ou sous forme de précurseur.

2. Système thérapeutique selon la revendication 1, caractérisé en ce qu'il dégage entre 0,05 et 5,0 microgrammes de CALCIPOTRIOL par cm² et par 24 heures.

3. Système thérapeutique selon la revendication 1 ou 2, caractérisé en ce qu'il contient une feuille polymère très flexible et perméable à la vapeur d'eau, à titre de couche dorsale.

4. Système thérapeutique selon la revendication 3, caractérisé en ce que le polymère est un polyuréthanne.

5. Système thérapeutique selon une ou plusieurs des revendications 1 à 4, caractérisé en ce que le réservoir de principe actif est formé à l'aide d'une couche de gel ou d'une couche de colloïde.

6. Système thérapeutique selon une ou plusieurs des revendications 1 à 5, caractérisé en ce que la diffusion du principe actif hors du réservoir ou la perméation du principe actif dans la peau peuvent être influencées par l'addition de substances appropriées.

7. Système thérapeutique selon une ou plusieurs des revendications 1 à 6, caractérisé en ce qu'on met en oeuvre une combinaison de Calcipotriol, respectivement d'un précurseur du Calcipotriol avec d'autres principes actifs.

8. Système thérapeutique selon la revendication 7, caractérisé en ce qu'est présente une combinaison du principe actif avec de la cortisone, avec de l'alpha-tocophérol, avec de l'acide acétylsalicylique et/ou avec de l'acide fumarique.

9. Procédé pour la préparation d'un système thérapeutique selon une ou plusieurs des revendications 1 à 8, caractérisé en ce qu'on introduit le CALCIPOTRIOL, respectivement son précurseur sous forme solide, en solution ou en dispersion dans le système d'administration, des adjuvants habituels pouvant être ajoutés.

10. Utilisation d'un système thérapeutique selon une ou plusieurs des revendications 1 à 9 pour la préparation d'un médicament prêt à l'emploi pour la thérapie de maladies cutanées liées à une forte prolifération cellulaire, en particulier le psoriasis.
